Europäisches Patentamt

European Patent Office

Office européen des brevets

(19)

(11) Veröffentlichungsnummer: **0 133 583**

**A1**

(12) ## EUROPÄISCHE PATENTANMELDUNG

(21) Anmeldenummer: 84109543.3

(22) Anmeldetag: 10.08.84

(51) Int. Cl.⁴: **A 61 B 5/02**
**A 61 M 5/14**

(30) Priorität: 12.08.83 DE 3329167
09.03.84 CH 1173/84

(43) Veröffentlichungstag der Anmeldung:
27.02.85 Patentblatt 85/9

(84) Benannte Vertragsstaaten:
AT BE CH DE FR GB IT LI LU NL SE

(71) Anmelder: Sterimed Gesellschaft für medizinischen
Bedarf mbH
Fasanerieweg 15
D-6600 Saarbrücken(DE)

(72) Erfinder: Kramann, Bernhard, Prof. Dr.
Preysingstrasse 11
D-8000 München 51(DE)

(74) Vertreter: Suchy, Herbert, Dr.
Byk Gulden Lomberg Chemische Fabrik GmbH
Byk-Gulden-Strasse 2 Postfach 6500
D-7750 Konstanz(DE)

(54) **Infusionsanordnung mit einer Vorrichtung zur Bestimmung des zentralvenösen Blutdrucks.**

(57) Infusionsanordnung mit einer Vorrichtung zur Bestimmung des zentralvenösen Drucks, umfassend eine Infusionsflasche (2) und eine Infusionsleitung (4), in die eine Druckabnehmerkammer (7) und eine zwischen Infusionsflasche (2) und Druckabenehmerkammer angeordnete Abklemmvorrichtung (8) zwischengeschaltet sind.

Die Druckabnehmerkammer (7) enthält eine erste leicht verformbare Kammer (27) und eine zweite leicht verformbare Kammer (16), wobei die erste Kammer (27) mit der Infusionsleitung (4) und mit einem Venenkatheter (5) und die zweite Kammer (16) über eine Schlauchleitung (14) mit dem Steigrohr (13) verbunden ist.

EP 0 133 583 A1

./...

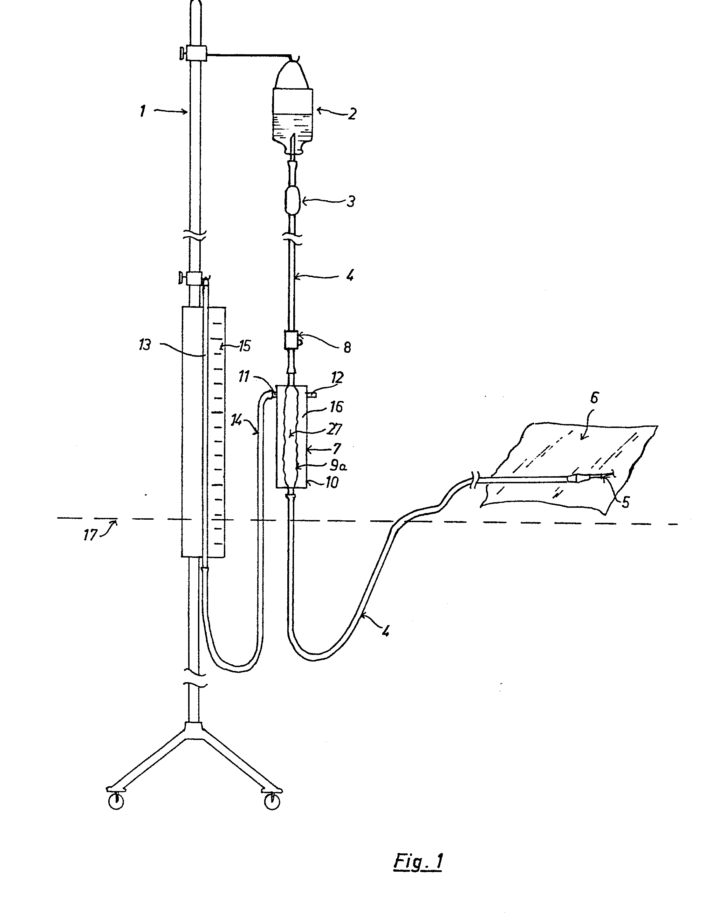

<u>Fig. 1</u>

## Infusionsanordnung mit einer Vorrichtung zur Bestimmung des zentral-venösen Blutdrucks

### Technisches Gebiet

Die Erfindung bezieht sich auf eine Infusionsanordnung mit einer Vorrichtung zur Bestimmung des zentralvenösen Druckes, umfassend eine Infusionsflasche und eine Infusionsleitung, in die eine Druckabnehmerkammer und eine zwischen Infusionsflasche und Druckabnehmerkammer angeordnete Abklemmvorrichtung zwischengeschaltet sind, sowie ein über eine Schlauchleitung mit der Druckabnehmerkammer in Verbindung stehendes Steigrohr.

### Stand der Technik

Der Bestimmung des zentralvenösen Druckes (ZVD) kommt bei kritischen Zuständen wie während und nach chirurgischen Operationen sowie bei der Behandlung von der Intensivpflege bedürftigen Verletzten eine große Bedeutung zu.

Ein weitverbreitetes Verfahren besteht darin, an eine von einer Infusionsflasche zu einem ins zentrale Venensystem reichenden Katheter füh-rende Schlauchleitung im Nebenschluß über einen Mehrwegehahn ein Manometersteigrohr anzuschliessen. Zur Messung des ZVD wird der Mehrwegehahn in eine Stellung gebracht, in der die Zufuhr von Infusionsflüssigkeit unterbrochen ist, jedoch das Manometersteigrohr über die zum Venenkatheter führende Schlauchleitung mit dem zentralen Venensystem in Verbindung steht. Der ZVD kann dann direkt aus der Steighöhe im Manometersteigrohr oberhalb eines übereinkömmlich patientenbezogenen Nullniveaus, wie vor allem der Höhe des rechten Herzvorhofs, abgelesen werden.

Es wurden verschiedene Vorschläge gemacht, um die auf der Hand liegenden Nachteile zu vermeiden: Die Nachteile sind durch das Vorhandensein einer stagnierenden Flüssigkeitssäule gegeben, die Anschluß an den zentralen Venenkatheter hat. Eine über viele Stunden stagnierende Flüssigkeitssäule beinhaltet immer die Gefahr der Infektion. Durch Rückfluß
des in das Manometerrohr eindringenden Blutes kann die Infektionsgefahr
verstärkt werden und kleine Thrombenbildungen können die Öffnungen des
Dreiwegehahnes verstopfen. Die Kontamination mit Keimen kann über den
Dreiwegehahn oder über den Luftzutritt am Ende der Flüssigkeitssäule
erfolgen. Darüberhinaus geschieht es beim praktischen Gebrauch der herkömmlichen Meßsysteme nicht selten, daß durch die fehlerhafte Einstellung des Dreiwegehahns unbemerkt Flüssigkeit vom Infusionssystem über
das Manometerrohr ins Freie überläuft und damit verlorengeht. Auf diese
Weise kann es zu fehlerhafter Bestimmung der Flüssigkeitsbilanz kommen.

In der US-PS 4 300 572 wird ein geschlossenes System vorgeschlagen, bei
dem ein Teil der Verbindungsleitung von der Tropfflasche zum Venenkatheter als Manometersteigrohr ausgebildet ist. Das Manometersteigrohr
ist am oberen Ende und unteren Ende jeweils mit einem Zweiwegehahn versehen. Der obere Zweiwegehahn erlaubt es, das Manometersteigrohr wahlweise mit der Infusionsflasche (Stellung I) oder einem mit steriler
Luft gefüllten Luftsack (Stellung II) zu verbinden. Der untere Zweiwegehahn stellt wahlweise eine Verbindung des Manometersteigrohrs zum Venenkatheter (Stellung I) oder zu einem geschlossenen Abfallbehälter
(Stellung II) her. Während der Infusion stehen beide Zweiwegehähne in
Stellung I. Zur Bestimmung des ZVD wird der obere Zweiwegehahn in Stellung II gebracht. Zum Umstellen auf Infusion wird der untere Zweiwegehahn in Stellung I gebracht. Sobald alle Luft aus dem Manometersteigrohr in den Abfallbehälter gespült ist, wird der untere Zweiwegehahn
wieder in Stellung I gebracht. Nachteilig an diesem Vorschlag erscheint
der relativ große Aufwand und die Gefahr, daß bei Fehlbedienung Luft in
das Blutgefäßsystem des Patienten gelangt.

In der DE-OS 26 30 974 wird ein geschlossenes System vorgeschlagen, bei
dem in den Verbindungsschlauch eine durchsichtige Kammer zwischengeschaltet ist. In der Kammer befindet sich eine zusammendrückbare Blase,
deren Innenraum zur Atmosphäre entlüftet ist. Zur Bestimmung des ZVD
soll der Schlauch oberhalb der Kammer abgeklemmt und die Kammer von
Hand soweit angehoben werden, bis sich die Blase plötzlich aufbläht.

Der ZVD ergibt sich dann aus der Höhe der Kammer über dem patientenbezogenen Nullniveau. Nachteilig an diesem Vorschlag erscheint, daß es für den Untersucher ziemlich schwierig ist, bei den atemabhängigen Schwankungen des ZVD die richtige Höhe der Kammer zu ermitteln, bei der ein Gleichgewicht zwischen Fluiddruck und atmosphärischem Druck erreicht ist. Hinzu kommt, daß bei Änderungen des ZVD der neue Wert erst ermittelt werden kann, nachdem die Kammer wieder von Hand auf das neue Ausgleichsniveau gebracht wurde.

## Beschreibung der Erfindung

Die Aufgabe der vorliegenden Erfindung bestand darin, eine Infusionsanordnung mit Vorrichtung zur Bestimmung des ZVD, umfassend eine Infusionsflasche und eine Infusionsleitung, in die eine Druckabnehmerkammer und eine zwischen Infusionsflasche und Druckabnehmerkammer angeordnete Abklemmvorrichtung zwischengeschaltet sind, sowie ein über eine Schlauchleitung mit der Druckabnehmerkammer in Verbindung stehendes Steigrohr, zur Verfügung zu stellen, das einfach aufgebaut ist, Fehlbedienungen ausschließt, bakterielle Verseuchung und Thrombenbildung vermeidet und auch eine stetige Beobachtung des ZVD zuläßt.

Überraschend einfach wird diese Aufgabe erfindungsgemäß dadurch gelöst, daß die Druckabnehmerkammer eine erste leicht verformbare Kammer und eine zweite leicht verformbare Kammer enthält, wobei die erste Kammer mit der Infusionsleitung und mit dem Venenkatheter und die zweite Kammer mit dem Steigrohr verbunden ist.

Bevorzugte Ausführungsformen ergeben sich aus den Unteransprüchen.

Die Anwendung der erfindungsgemäßen Vorrichtung erfolgt so, daß man die Infusionsflasche wie üblich an einem herkömmlichen Stativ aufhängt und über die erste Kammer der Druckabnehmerkammer mit dem in das Venensystem des Patienten eingelegten Venenkatheter verbindet. Das Steigrohr wird mit der zweiten Kammer der Druckabnehmerkammer verbunden. Dann wird die zweite Kammer der Druckabnehmerkammer über das Steigrohr oder

S34 EP                          -4-                      0133583

besser über einen freien Anschlußstutzen mit Flüssigkeit so weit gefüllt, daß die Menge der Flüssigkeit auch bei minimalem Volumen der
ersten Kammer ausreicht, um im Steigrohr die maximal zu erwartende
Druckhöhe über dem patientenbezogenen Nullniveau anzeigen zu können.
Für die Messung des ZVD wird die Abklemmvorrichtung in der Infusionsleitung geschlossen.

Das Steigrohr ist zweckmäßigerweise am Infusionsstativ aufgehängt und
mit einer Skala hinterlegt. Es ist entweder als starres Rohr oder auch
als Schlauch ausgebildet. Anstatt den zentralvenösen Druck als Steighöhe der Wassersäule über dem patientenbezogenen Nullniveau abzulesen,
kann der Druck auch aus der Höhe des Nullniveaus mit Hilfe eines Drucksensors abgenommen werden und auf übliche Weise entweder analog oder
digital angezeigt werden. Bei der Bestimmung des Druckes mittels Drucksensor können die jeweils gemessenen Werte auch auf einem Blattschreiber ausgegeben werden und/oder einer Alarmeinrichtung zugeführt werden,
die bei kritischen zentralvenösen Drücken Alarmsignale abgibt. Die Höhe, in welcher die Druckabnehmerkammer innerhalb der Infusionsleitung
zwischengeschaltet ist, ist beliebig, jedoch ist sie vorzugsweise auf
einem Niveau zwischen der maximal zu erwartenden Druckhöhe und dem patientenbezogenen Nullniveau angebracht. Die zwischen Druckabnehmerkammer und Infusionsflasche vorgesehene Abklemmvorrichtung ist eine herkömmliche Schlauchklemme, wie z.B. eine Rollklemme oder dergleichen.
Wenn die Unterteilung der Druckabnehmerkammer in zwei Kammern durch
eine leicht verformbare Zwischenwand erfolgt, so ist diese so bemessen,
daß sich ihr Volumen ohne Dehnbeanspruchung mindestens um so viel ändern kann, wie es dem Innenvolumen des Steigrohrs zwischen maximal und
minimal zu erwartender Druckhöhe entspricht. Es ist bekannt daß der ZVD
sich etwa im Bereich von minus 5 bis plus 35 cm Wassersäule bewegen
kann. Die Anschlußstutzen der Druckabnehmerkammer ebenso wie die Anschlüsse der Infusionsleitung an die erste Kammer können beliebig gestaltet sein, jedoch sind die in der Medizintechnik üblichen genormten
Anschlüsse, wie z.B. Luer-Lock bevorzugt.

Wenn eine Kammer als Folienschlauch ausgebildet ist, so versteht es
sich von selbst, daß alternativ das Innere des Folienschlauches oder
der Zwischenraum zwischen Folienschlauch und Wand der Druckabnehmerkammer als erste Kammer der Druckabnehmerkammer verwendet werden kann.
Ebenso ist es selbstverständlich, die erfindungsgemäße Vorrichtung ohne
Infusionsflasche, d.h. nur zur Druckanzeige zu verwenden. Es ist auch
leicht einzusehen, daß die Druckabnehmerkammer nicht auf eine rohrförmige äußere Gestalt beschränkt ist. Die Herstellung der erfindungsgemäßen Vorrichtung ist billig, so daß sie als Einmalartikel verwendet
werden kann, womit die mit einer Resterilisation verbundenen Probleme
vermieden werden.

Die beiden Kammern der Druckabnehmerkammer können in einer erfindungsgemäßen Ausführungsform als kollabierbare Folienschläuche gebildet
sein. Die Durchmesser der beiden Folienschläuche sind jeweils so bemessen, daß sie in gefülltem Zustand den gesamten Innenraum der Druckabnehmerkammer ausfüllen.

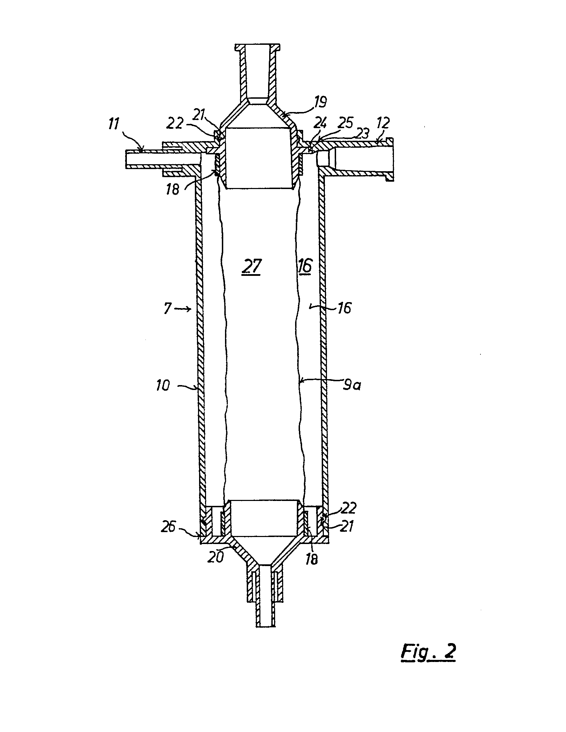

Nachfolgend wird die erfindungsgemäße Vorrichtung anhand von Fig. 1, 2
3 und 4 näher erläutert.

Fig. 1 zeigt in schematischer Darstellung ein Infusionsgerät mit der
        erfindungsgemäßen Vorrichtung zur Bestimmung des ZVD.

Fig. 2 zeigt einen Querschnitt durch eine Ausführungsform einer Druckabnehmerkammer.

Fig. 3 und 4 zeigen in schematischer Darstellung Querschnitte durch
        Ausführungsformen der Druckabnehmerkammer.

In Fig. 1 ist eine an einem Infusionsstativ 1 hängende Infusionsflasche
2, die über eine Tropfkammer 3 über die Infusionsleitung 4 mit einem
Venenkatheter 5 verbunden ist, zu erkennen. Der Venenkatheter 5 ist in
das Venensystem des Patienten 6 eingeschoben. In die Infusionsleitung 4
ist die Druckabnehmerkammer 7 zwischengeschaltet. Oberhalb der Druckabnehmerkammer 7 befindet sich auf der Infusionsleitung 4 eine Abklemmvorrichtung 8, z.B. in Gestalt einer üblichen Rollklemme, mit deren
Hilfe der Querschnitt der Infusionsleitung 4 verschlossen werden kann.
Die Druckabnehmerkammer 7 umschließt einen die leicht verformbare Zwischenwand bildenden kollabierbaren Folienschlauch 9a, dessen Inneres hier die erste Kammer 27 darstellt. Die Wand 10 der Druckabnehmerkammer 7 ist beispielsweise als Rohr ausgebildet. Sie weist zwei Anschlußstutzen 11, 12 für die zweite Kammer 16 auf. An den ersten Anschlußstutzen 11 ist eine zum Steigrohr 13 führende Schlauchleitung 14 angeschlossen. Das Steigrohr 13 ist mit einer Meßskala 15 hinterlegt. Die Meßskala 15 kann am Infusionsstativ 1 befestigt werden. Das Steigrohr 13 ist entweder als starres durchsichtiges Rohr aus Glas oder Kunststoff oder einfach als Schlauch aus durchsichtigem oder zumindest durchscheinendem Material ausgeführt. Um die Ablesung zu erleichtern kann die Anzeigeflüssigkeit angefärbt sein. Der zweite Anschlußstutzen 12 der Druckabnehmerkammer 7 dient zum Einfüllen der Meßflüssigkeit in die zweite Kammer 16, d.h. den Zwischenraum zwischen Folienschlauch 9a und Wand 10, und wird nach dem Einfüllen der Meßflüssigkeit beispielsweise durch einen Stopfen verschlossen. Das patientenbezogene Nullniveau, das z.B. der Höhe des rechten Herzvorhofs entspricht, ist durch die gestrichelte Linie 17 angedeutet.

Aus Fig. 2 ist der Aufbau einer besonderen Ausführungsform einer Druckabnehmerkammer 7 zu entnehmen. Die Wand 10 ist rohrförmig und mit einem Anschlußstutzen 11 zum Aufstecken der zum Steigrohr 13 führenden Schlauchleitung 14 (siehe Fig. 1) ausgestattet. Gegenüber liegt der Anschlußstutzen 12, der hier einen Innenkonus zur Aufnahme des Ansatzes, beispielsweise einer mit Flüssigkeit gefüllten Spritze, aufweist. Der Anschlußstutzen 12 wird mit einem passenden Stopfen verschlossen. Der Folienschlauch 9a ist mittels Schellen 18 auf dem oberen Ansatzstück 19 und dem unteren Ansatzstück 20 befestigt. Die beiden Ansatzstücke 19, 20 sind jeweils mit der Wand 10 fest verbunden, beispielsweise durch außenseitige Ringwulste 21, die in entsprechenden Sicken 22 der Wand 10 einrasten. Ein ringförmiger Flansch 23 des oberen Ansatzstückes liegt in einer entsprechenden ringförmigen Ausnehmung 24 der Wand 10 an.

Zur Montage der Druckabnehmerkammer 7 wird zuerst der Folienschlauch 9a auf die beiden Anschlußstücke 19, 20 aufgezogen und mittels Schellen 18 hieran fixiert. Das obere Ansatzstück 19 wird sodann von unten durch die rohrförmige Wand 10 hindurchgeführt und an dessen oberem Ende 25 durch Einrasten fixiert. Sodann wird das untere Ansatzstück 20 in das

untere Ende 26 der rohrförmigen Wand 10 eingeführt und eingerastet. Gewünschtenfalls können die Ansatzstücke 19, 20 zusätzlich mit der Wand 10 verklebt werden.

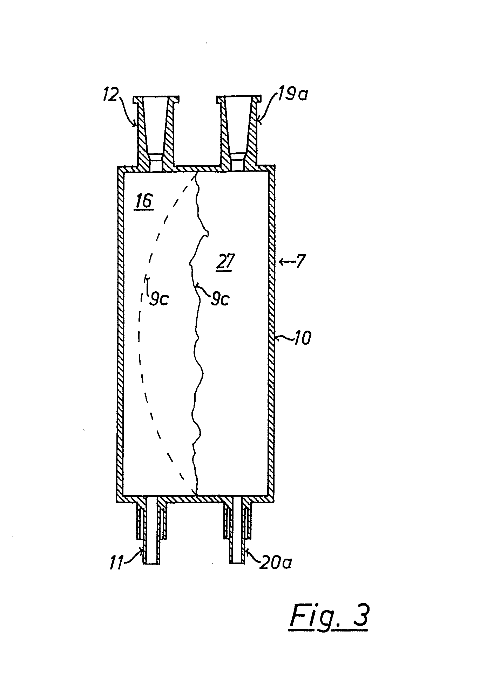

In der in Fig. 3 schematisch im Querschnitt dargestellten Druckabnehmerkammer 7 werden die erste Kammer 27 und die zweite Kammer 16 durch eine locker aufgehängte Membran 9c flüssigkeitsdicht voneinander getrennt. Durch die lockere Aufhängung der Membran 9c ist gewährleistet, daß sie sich beim Druckausgleich in beide Richtungen auswölben kann, ohne daß die Membran 9c gedehnt werden muß. Zur Verdeutlichung ist die Membran 9c zusätzlich gestrichelt in ihrer aus der Gleichgewichtslage nach links ausgelenkten Stellung gezeigt. Um ein Übertreten von Flüssigkeit von der ersten Kammer 27 in die zweite Kammer 16 und umgekehrt sicher zu vermeiden, kann die Membran 9c auch doppelt ausgeführt sein. An den Anschlußstutzen 11 wird die zum Steigrohr 13 (Fig.1) führende Schlauchleitung 14 (Fig. 1) angeschlossen. Der Anschlußstutzen 12 dient zum Einfüllen der Meßflüssigkeit in die zweite Kammer 16. Mittels der Anschlußstutzen 19a und 20a wird die Druckabnehmerkammer 7 in die Infusionsleitung 4 zwischengeschaltet.

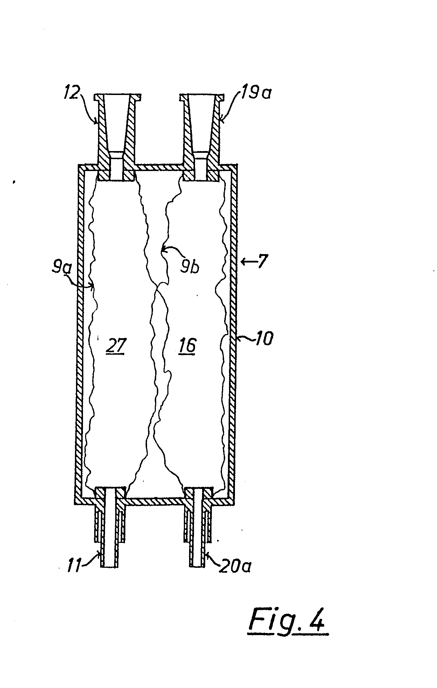

In Fig. 4 ist eine Variante der in Fig. 3 dargestellten Druckabnehmerkammer 7 gezeigt. Sowohl die erste Kammer 27 als auch die zweite Kammer 16 sind durch kollabierbare Folienschläuche 9a und 9b gebildet. Durch die Ausführung beider Kammern 27, 16 als Folienschlauch 9a, 9b ist auch hier eine erhöhte Sicherheit gegen ein Übertreten von Flüssigkeit zwischen den beiden Kammern gegeben.

<u>P a t e n t a n s p r ü c h e</u>

1.    Infusionsanordnung mit einer Vorrichtung zur Bestimmung des zentralvenösen Drucks, umfassend eine Infusionsflasche und eine Infusionsleitung, in die eine Druckabnehmerkammer und eine zwischen Infusionsflasche und Druckabnehmerkammer angeordnete Abklemmvorrichtung zwischengeschaltet sind, sowie ein über eine Schlauchleitung mit der Druckabnehmerkammer in Verbindung stehendes Steigrohr, dadurch gekennzeichnet, daß die Druckabnehmerkammer (7) eine erste leicht verformbare Kammer (27) und eine zweite leicht verformbare Kammer (16) enthält, wobei die erste Kammer (27) mit der Infusionsleitung (4) und mit dem Venenkatheter und die zweite Kammer (16) mit dem Steigrohr (13) verbunden ist.

2.    Infusionsanordnung nach Anspruch 1, dadurch gekennzeichnet, daß die Druckabnehmerkammer (7) rohrförmig ausgebildet ist. (Fig. 2)

3.    Infusionsanordnung nach Anspruch 1, dadurch gekennzeichnet, daß die erste Kammer (27) und die zweite Kammer (16) durch eine leicht verformbare membranartige Zwischenwand (9c) in der Druckabnehmerkammer (7) gebildet sind. (Fig. 3)

4.    Infusionsanordnung nach Anspruch 1, dadurch gekennzeichnet, daß eine der Kammern (27, 16) durch einen kollabierbaren Folienschlauch (9a) gebildet ist. (Fig. 2)

5.    Infusionsanordnung nach Anspruch 1, dadurch gekennzeichnet, daß beide Kammern (27, 16) jeweils durch einen kollabierbaren Folienschlauch (9a, 9b) gebildet sind. (Fig. 4)

6.    Infusionsanordnung nach Anspruch 4, dadurch gekennzeichnet, daß die Enden des Folienschlauches (9a) auf zwei die Druckabnehmerkammer (7) dicht abschließenden Ansatzstücke (19, 20) aufgezogen sind. (Fig. 2)

7.    Infusionsanordnung nach Anspruch 6, dadurch gekennzeichnet, daß

S34 EP  -9-  0133583

die Enden des Folienschlauches (9a) mittels Schellen (18) an den beiden
Ansatzstücken (19, 20) befestigt sind. (Fig. 2)

_Fig. 1_

_Fig. 2_

19a 7 10 27 9c 16 9c 12 20a 11

*Fig. 3*

_Fig.4_

0133583

# EUROPÄISCHER RECHERCHENBERICHT

**0133583**
Nummer der Anmeldung

EP 84 10 9543

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | WO-A-8 202 657 (M. EBERT) * Zusammenfassung; Seite 8, Zeile 13 - Seite 9, Zeile 13; Seite 10, Zeilen 1-17; Seite 13, Zeilen 4-12; Abbildungen 1-6 * | 1,3 | A 61 B 5/02 A 61 M 5/14 |
| | --- | | |
| A | US-A-4 146 028 (R.L. LEFEVRE) * Zusammenfassung; Spalte 2, Zeile 34 - Spalte 3, Zeile 1; Abbildungen 1-3 * | 1-4,6 | |
| | --- | | |
| A | US-A-4 386 929 (J.R. PEERY) * Zusammenfassung; Spalte 1, Zeile 67 - Spalte 2, Zeile 16; Spalte 2, Zeile 65 - Spalte 3, Zeile 5; Abbildungen 1-3 * | 2-4,6, 7 | |
| | --- | | RECHERCHIERTE SACHGEBIETE (Int. Cl.4) |
| A | US-A-2 976 865 (R.E. SHIPLEY) * Spalte 3, Zeilen 8-48; Abbildung 1 * | 2,3 | A 61 B A 61 M |
| | --- | | |
| A | GB-A-1 116 997 (CANADAIR LTD.) * Seite 2, Zeilen 88-124; Seite 3, Zeilen 5-49; Seite 3, Zeile 124 - Seite 4, Zeile 16; Abbildungen 1,5 * | 1,3 | |
| | --- | | |
| D,A | US-A-4 300 572 (D.R. KNIGHTON) | | |
| | --- -/- | | |

Der vorliegende Recherchenbericht wurde für alle Patentansprüche erstellt.

| Recherchenort DEN HAAG | Abschlußdatum der Recherche 01-11-1984 | Prüfer RIEB K.D. |
|---|---|---|

## EUROPÄISCHER RECHERCHENBERICHT

Europäisches Patentamt

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,A | DE-A-2 630 974 (AMERICAN HOSPITAL SUPPLY CORP.)  ----- | | |

RECHERCHIERTE SACHGEBIETE (Int Cl 4)

Der vorliegende Recherchenbericht wurde für alle Patentansprüche erstellt.

| Recherchenort DEN HAAG | Abschlußdatum der Recherche 01-11-1984 | Prüfer RIEB K.D. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82